# EUROPÄISCHE PATENTSCHRIFT

(11) **EP 3 784 308 B1**
(45) Veröffentlichungstag und Bekanntmachung des Hinweises auf die Patenterteilung: **28.02.2024**
(21) Anmeldenummer: 19718688.5
(22) Anmeldetag: 17.04.2019
(51) Int. Cl.: A61M 1/16

(54) **DIALYSEGERÄT MIT BLUTLECKSENSOR**
DIALYSIS DEVICE WITH BLOOD LEAKAGE SENSOR
APPAREIL DE DIALYSE COMPRENANT UN CAPTEUR DE FUITE DE SANG

(30) Priorität: 23.04.2018 DE 102018109702
(43) Veröffentlichungstag der Anmeldung: 03.03.2021
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE)
(74) Vertreter: Herrmann, Uwe
(86) Internationale Anmeldenummer: PCT/EP2019/059972
(87) Internationale Veröffentlichungsnummer: WO 2019/206773

(56) Entgegenhaltungen:
- US-A- 4 181 610
- US-A- 5 690 821
- US-A1- 2016 058 933

## Beschreibung

Die Erfindung betrifft ein Dialysegerät mit einem Dialysator, einem extrakorporalen Blutkreislauf, einem Dialyselösungskreislauf und einer Steuereinheit.

Im Rahmen einer Dialysebehandlung zur Nierenersatztherapie wird Blut in einem extrakorporalen Blutkreislauf durch einen Dialysator geleitet, in dem es über eine semipermeable Membran mit einer Dialyselösung in Kontakt tritt. Die Membran besitzt Poren, die eine Diffusion kleiner Moleküle (Elektrolyte, Harnstoff) vom Blut in die Dialyselösung zulassen, wobei jedoch große Moleküle wie Eiweiße und Blutzellen zurückgehalten werden.

Im Falle einer Beschädigung in der semipermeablen Membran kann es zu einem unerwünschten Bluteintrag in die Dialyselösung kommen. Um einen solchen Fehler erkennen zu können, ist es im Stand der Technik bekannt, im Dialyselösungskreislauf stromabwärts des Dialysators einen Sensor anzuordnen, mit dem das Vorhandensein von Blut in der vom Dialysator abfließenden Dialyselösung erkannt werden kann. Bekannte Beispiele umfassen optische Sensoren, die eine Extinktionsmessung durchführen. Zeigt das Sensorsignal eine bestimmte Auffälligkeit, löst die Steuereinheit des Geräts einen Alarm aus und/oder bewirkt einen sofortigen Pumpenstopp.

Aus der US5690821A sind beispielsweise eine Beladungsplattform und ein Additionssystem für Chemikalien für eine Dialysataufbereitungsanlage bekannt. Die US4181610A offenbart einen Blutlecksensor für extrakorporale Blutbehandlungsmaschinen. Weiterhin ist ein Prozessor für eine extrakorporale Blutbehandlungsmaschine aus der US2016/058933A1 bekannt.

Ein Nachteil bestehender derartiger Lösungen liegt darin, dass es zu Fehlauslösungen aufgrund eines Lufteintrags in die Dialyselösung kommen kann (z.B. durch Nachentgasung oder schlechte Entlüftung des Dialysators).

Aufgabe der Erfindung ist es, ein Konzept bereitzustellen, um die Anzahl derartiger Fehlauslösungen zu verringern oder diese bestenfalls insgesamt zu vermeiden.

Die Erfindung wird durch die Merkmale des unabhängigen Anspruchs 1 definiert.

Vor diesem Hintergrund betrifft die Erfindung ein Dialysegerät mit einem Dialysator einem extrakorporalen Blutkreislauf, einem Dialyselösungskreislauf und einer Steuereinheit, wobei im Dialyselösungskreislauf stromabwärts des Dialysators ein Blutlecksensor angeordnet ist, wobei die Steuereinheit ausgebildet ist, eine Fehlerroutine einzuleiten, wenn das Signal des Blutlecksensors einen Hinweis auf ein vermeintliches Blutleck zeigt, wobei im Dialyselösungskreislauf stromabwärts des Dialysators ferner ein weiterer Sensor angeordnet ist, und wobei die Steuereinheit ausgebildet ist, das zeitlich mit dem Signal des Blutlecksensors korrelierte Signal des weiteren Sensors als Unterdrückungskriterium für die Einleitung der Fehlerroutine heranzuziehen.

Erfindungsgemäß wird also die Fehlerroutine dann zumindest temporär unterdrückt, wenn in zeitlicher Korrelation auch das Signal des weiteren Sensors eine bestimmte Auffälligkeit zeigt, welche auf einen Störfaktor wie beispielsweise einen Lufteintrag schließen lässt. Der Hinweis auf ein vermeintliches Blutleck bzw. die Auffälligkeit kann in einem Passieren eines Schwellenwertes liegen, also im Sinne einer Überschreitung einer oberen Grenze bzw. im Sinne einer Unterschreitung einer unteren Grenze.

Die Anordnung eines Blutlecksensors im Dialyselösungskreislauf und stromabwärts des Dialysators ist an sich bekannt. Der Sensor dient der Erkennung von Bluteinträgen in die vom Dialysator abfließende Dialyselösung. Die Erfindung beruht also auf der Idee, die Einleitung einer für einen Bluteintrag in die Dialyseflüssigkeit vorgesehenen Fehlerroutine nicht alleine davon abhängig zu machen, ob das Signal des Blutlecksensors einen Hinweis auf ein vermeintliches Blutleck zeigt, sondern weitere Messwerte abzufragen, die für die Dialyseflüssigkeit stromabwärts des Dialysators ermittelt werden. So kann das Risiko einer Fehlauslösung verringert werden.

In einer Ausführungsform ist vorgesehen, dass es sich bei dem weiteren Sensor um einen Sensor handelt, der sich eines vom Blutlecksensor unterschiedlichen Messverfahrens bedient und/oder der einen vom Blutlecksensor unterschiedlichen Messparameter erfasst, wobei vorzugsweise vorgesehen ist, dass der Blutlecksensor ausgebildet ist. So wird sichergestellt, dass das Signal des weiteren Sensors eine tatsächliche Kontrolle darstellt und nicht einfach denselben Hinweis dupliziert.

In einer Ausführungsform ist vorgesehen, dass es sich bei dem weiteren Sensor um einen Sensor handelt, dessen Signal durch Lufteinträge in die Dialyseflüssigkeit beeinflusst wird und dessen Signal durch Bluteinträge in die Dialyseflüssigkeit nicht oder weniger stark beeinflusst wird. Lufteinträge in die verbrauchte Dialyseflüssigkeit, welche beispielsweise durch Nachentgasung oder schlechte Entlüftung des Dialysators hervorgerufen werden, stellen eine mögliche Ursache für falsch positive Signale des Blutlecksensors dar. Anhand von Sensorsignalen, welche Lufteinträge erkennen und von Blutsignalen unterscheiden können, kann daher eine wirkungsvolle Überprüfung stattfinden und Fehler können vermieden werden.

In einer Ausführungsform ist vorgesehen, dass es sich bei dem weiteren Sensor um einen Leitfähigkeitssensor handelt. Die Anordnung eines stromabwärts des Dialysators im Dialyseflüssigkeitskreislauf angeordneten Leitfähigkeitssensors ist an sich bekannt, insbesondere im Zusammenhang mit der Messung der Dialysedosis (Online Clearance Measurement, OCM). Diese Messung umfasst einen zeitkorrigierten Vergleich der Leitfähigkeiten der Dialyseflüssigkeit stromaufwärts und stromabwärts des Dialysators, wobei angenommen wird, dass die Durchlässigkeit der Membran für Harnstoff in einem festen Verhältnis zu deren Durchlässigkeit für Elektrolyte steht.

In einer Ausführungsform ist vorgesehen, dass stromabwärts des Dialysators ein Luftabscheider angeordnet ist und dass es sich bei dem weiteren Sensor um einen an dem Luftabscheider angeordneten Luftdetektor, vorzugsweise Levelsensor handelt. Auch die Anordnung eines Luftabscheiders stromabwärts des Dialysators im Dialyselösungskreislauf ist an sich bekannt. Durch einen Luftabscheider soll vermieden werden, dass die Genauigkeit der volumetrischen Bilanzierung der Dialyseflüssigkeit, welche zur Bestimmung und Regelung der dem Patienten entzogenen Flüssigkeitsmenge erforderlich ist, nicht durch Lufteinträge in die Dialyseflüssigkeit beeinträchtigt wird. Bei den Lufteinträgen, welche durch den stromabwärts des Dialysators angeordneten Luftabscheider entfernt werden sollen, handelt es sich um genau diejenigen, beispielsweise durch Nachentgasung oder schlechte Entlüftung des Dialysators hervorgerufenen Lufteinträge, welche auch für Fehlsignale am Blutlecksensor verantwortlich sein können.

Die Signale des Luftdetektors oder Leitfähigkeitssensors eignen sich einerseits deshalb besonders gut als Unterdrückungskriterium im Sinne der Erfindung, weil Sensoren verwendet werden, welche bereits aus anderen Gründen ohnehin im Dialyseflüssigkeitskreislauf vorhanden sein können, sodass kein apparativer Mehraufwand entsteht. Ferner eignen sich diese Messwerte besonders gut, da Lufteinträge und Bluteinträge an diesen Sensoren unterschiedliche Reaktionen hervorrufen. So führt ein Lufteintrag in der Regel zu einem Einbruch des Leitfähigkeitssignals, während ein Bluteintrag die Leitfähigkeit der Dialyseflüssigkeit aufgrund des nominell identischen Elektrolytgehalts kaum beeinflussen sollte. Das Signal des Luftdetektors bleibt von einem Bluteintrag gänzlich unbeeinflusst, während ein Lufteintrag zu einer Levelverschiebung am Luftabscheider führt.

Erfindungsgemäß ist vorgesehen, dass im Dialyselösungskreislauf stromabwärts des Dialysators ein Luftabscheider angeordnet ist und dass zwei weitere Sensoren vorgesehen sind, nämlich ein Leitfähigkeitssensor sowie ein an dem Luftabscheider angeordneter Luftdetektor, vorzugsweise Levelsensor, wobei die Steuereinheit ausgebildet ist, die zeitlich korrelierten Signale beider dieser weiteren Sensoren als Unterdrückungskriterien für die Einleitung der Fehlerroutine heranzuziehen. Durch die Heranziehung beider dieser Informationen kann eine besonders deutliche Reduktion der Fehlauslösungen der Fehlerroutine erreicht werden.

In einer Ausführungsform ist vorgesehen, dass die zeitliche Korrelation anhand des Volumenflusses der Dialyseflüssigkeit und anhand der zwischen den entsprechenden Sensoren liegenden Kreislaufinnenvolumina ermittelt wird. Die zeitliche Korrelation bedeutet vorzugsweise also keine gleichzeitige Betrachtung der unterschiedlichen Messgrößen, sondern es wird berücksichtigt, dass die Dialyseflüssigkeit eine gewisse Zeit benötigt, um von einem Sensor zum weiteren Sensor zu fließen. Liegt beispielsweise zwischen einem Blutlecksensor und einem stromabwärts davon angeordneten Leitfähigkeitssensor ein Kreislaufinnenvolumen von v₁ cm³ und fließt die Dialyseflüssigkeit mit einem Volumenfluss von f₁ cm³/s, so verwendet die erfindungsgemäße Routine in dieser Ausführungsform das Leitfähigkeitssignal zum Zeitpunkt t+t₁ als Unterdrückungskriterium, wobei t dem Zeitpunkt des auffälligen Blutlecksignals und t₁ dem Korrelationszeitraum entspricht, der sich aus dem Quotienten v₁/f₁ ergibt.

In einer Ausführungsform ist vorgesehen, dass die Steuereinheit ausgebildet ist, die Fehlerroutine erst nach Ablauf einer Karenzzeit einzuleiten, während der die Auffälligkeit am Blutlecksensor mehrmals oder fortlaufend erkannt wird. Es kann also vorgesehen sein, dass zusätzlich eine an sich bekannte zeitliche Alarmunterdrückung stattfindet, wobei das Signal des Blutlecksensors über eine bestimmte Karenzzeit von beispielsweise mindestens zwei, mindestens fünf oder mindestens zehn Sekunden beobachtet und die Fehlerroutine nur dann eingeleitet wird, wenn die eingangs beobachtete Auffälligkeit während des Beobachtungszeitraums bestehen bleibt oder sich wiederholt.

In einer Ausführungsform ist vorgesehen, dass die Steuereinheit ausgebildet ist, das Signal des weiteren Sensors während der Karenzzeit mehrmals oder fortlaufend abzufragen und diese Messwerte als Unterdrückungskriterien heranzuziehen. Im Rahmen dieser Ausführungsform ist also vorgesehen, dass nicht nur das Signal des Blutlecksensors über eine bestimmte Karenzzeit beobachtet wird, sondern auch das Signal des oder der weiteren Sensoren. So kann eine weitere Reduzierung von Fehlauslösungen erreicht werden.

In einer Ausführungsform ist vorgesehen, dass die Steuereinheit ausgebildet ist, die Karenzzeit zu verlängern, wenn am weiteren Sensor ein Signal erkannt wird, welches auf einen Störfaktor schließen lässt. In dieser Ausführungsform erfolgt also keine endgültige Unterdrückung der Fehlerroutine, sondern lediglich eine Verlängerung der Karenzzeit. Dies beruht auf der Überlegung, dass ein Lufteintrag immer lediglich temporär sein und ein relativ abruptes Signalverhalten zeigen sollte, während ein Blutleck zu einer langsamer anlaufenden bzw. verschwindenden, länger währenden Einfärbung der verbrauchten Dialyseflüssigkeit führt. Wenn also tatsächlich ein Blutleck vorliegt, sollte sich nach einer verlängerten Karenzzeit ein Zustand einstellen, in welchem die Signale der weiteren Sensoren unauffällig bleiben.

In einer Ausführungsform ist vorgesehen, dass die Fehlerroutine die Ausgabe eines Alarmsignals umfasst. Geeignete Alarmsignale umfassen akustische oder optische Alarmsignale, die direkt an der Dialysemaschine und/oder an einem örtlich entfernten Überwachungsterminal ausgegeben werden können.

In einer Ausführungsform ist vorgesehen, dass die Fehlerroutine einen Behandlungsstopp umfasst. Ein Behandlungsstopp kann die Abschaltung einer im extrakorporalen Blutkreislauf angeordneten Blutpumpe und/oder einer im Dialyseflüssigkeitskreislauf angeordneten Dialyseflüssigkeitspumpe umfassen.

In einer Ausführungsform ist vorgesehen, dass es sich bei dem Blutlecksensor um einen optischen Sensor handelt, vorzugsweise um einen Sensor zur Durchführung einer Extinktionsmessung. Bei der Verwendung derartiger Sensoren kann es zu Fehlauslösungen durch Luftblasen kommen, da sowohl Bluteinträge als auch Lufteinträge typischerweise zu einer Signalschwächung führen, letzteres aufgrund von Streuung, Brechung, Reflexion etc. Daher ist eine Anwendung des erfindungsgemäßen Konzepts zur Alarmunterdrückung bei der Verwendung derartiger Sensoren besonders sinnvoll.

Als Dialysegerät wird im Rahmen der vorliegenden Erfindung ein Gerät zur Durchführung einer Hämodialyse, Hämodiafiltration oder Hämofiltration verstanden. Die Steuereinheit des Geräts steht mit den genannten Sensoren, also dem Blutlecksensor und dem oder den weiteren Sensoren in Verbindung. Auf der Steuereinheit ist ein Algorithmus hinterlegt, welcher die erfindungsgemäße Ausbildung der Steuereinheit zur Einleitung und gegebenenfalls Unterdrückung einer Fehlerroutine realisiert.

Anhand des erfindungsgemäßen Dialysegeräts können ein Dialyseverfahren sowie ein Verfahren zur Überwachung einer Dialysebehandlung realisiert werden, in welchem das zeitlich mit dem Signal des Blutlecksensors korrelierte Signal des weiteren Sensors als Unterdrückungskriterium für die Einleitung der Fehlerroutine herangezogen wird.

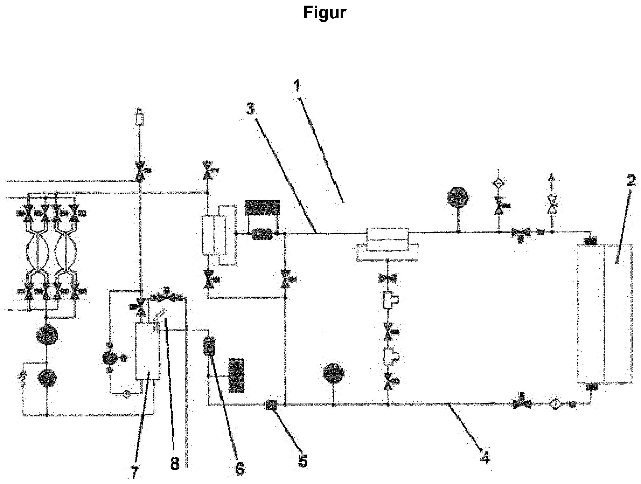

Weitere Einzelheiten und Vorteile der Erfindung ergeben sich aus dem nachfolgend anhand der Figur dargestellten Ausführungsbeispiel. Die einzige Figur zeigt ein Flussdiagramm eines Ausschnitts aus dem Dialyseflüssigkeitskreislaufs eines erfindungsgemäßen Dialysegeräts.

Das Flussdiagramm zeigt denjenigen Bereich des Dialyseflüssigkeitskreislaufs 1 eines erfindungsgemäß ausgebildeten Geräts, der sich nahe dem Dialysator 2 befindet. Die weiteren Bereiche des Dialyseflüssigkeitskreislaufs 1 sind für die Darstellung der vorliegenden Erfindung nicht relevant und wurden dementsprechend in der Darstellung ausgeklammert. Zudem beschränkt sich die nachfolgende Erläuterung auf die Nennung und Beschreibung derjenigen Elemente des Dialyseflüssigkeitskreislaufs 1, welche für das Verständnis der vorliegenden Erfindung wesentlich sind.

Der Dialyseflüssigkeitskreislauf 1 umfasst eine Vorlaufleitung 3, durch welche Dialyseflüssigkeit in den Dialysator 2 eingeleitet wird. Im Dialysator tritt die Dialyseflüssigkeit in Kontakt mit dem Blut des Patienten, welches anhand eines nicht dargestellten extrakorporalen Blutkreislaufs durch den Dialysator 2 geleitet wird. Der Kontakt erfolgt über eine semipermeable Membran mit kleinen Poren, welche eine Diffusion kleiner Moleküle wie Elektrolyte und Harnstoff zwischen Blut und Dialyselösung zulassen, jedoch große Moleküle wie Eiweiße und Blutzellen im Blut zurückhalten. Die verbrauchte Dialyselösung wird über die Rücklaufleitung 4 vom Dialysator 2 abgeleitet.

Durch Beschädigungen in der semipermeablen Membran des Dialysators kann Blut in die Dialyselösung übertreten, was bereits wegen einer Kontamination des Dialyseflüssigkeitskreislaufs zu vermeiden ist. Ferner kann es im Falle einer hohen Übertrittsrate auch zu einer Gefährdung des Patienten kommen, was selbstverständlich nicht zu tolerieren ist. Deshalb befindet sich in der Rücklaufleitung 4 ein optischer Blutlecksensor 5, der etwaiges in der verbrauchten Dialyselösung vorhandenes Blut durch optische Extinktionsmessung ermittelt. Sofern ein Hinweis auf ein vermeintliches Blutleck erkannt wird, kommt es zur Einleitung einer Fehlerroutine, welche eine optische und akustische Alarmausgabe umfasst und eine Notabschaltung der Behandlung, z.B. durch Stopp der Pumpen und/oder Schließen der Klammern einleitet.

Die Extinktionsmessung ist anfällig für Fehlauslösungen aufgrund von beispielsweise Lufteinschlüssen, welche unter anderem durch Streuungseffekte ebenfalls eine Abschwächung von durch die Dialyseflüssigkeit geleitetem Licht bewirken. Deshalb war im Stand der Technik bereits eine zeitliche Auslöseunterdrückung bekannt, bei welcher eine Fehlerroutine erst nach Ablauf einer bestimmten Karenzzeit ausgelöst wurde, in welcher die Auffälligkeit im Signal des Blutlecksensors wiederholt oder fortlaufend auftritt. Auch bei Anwendung einer solchen zeitlichen Auslöseunterdrückung kommt es aber zu nicht vernachlässigbaren Fehlauslösungen.

Die Erfindung macht es sich vor diesem Hintergrund zunutze, dass in der Rücklaufleitung 4 weitere Sensoren vorhanden sind, deren Messwerte durch das Vorhandensein von Luft beeinflusst werden und die durch das Vorhandensein von Blut nicht oder allenfalls geringfügig beeinflusst werden.

Nämlich umfasst das Dialysegerät einerseits einen in der Rücklaufleitung 4 angeordneten Leitfähigkeitssensor 6. Dieser Leitfähigkeitssensor 6 wirkt grundsätzlich an der Bestimmung der Dialysedosis im Rahmen eines Online Clearance Measurement ("OCM") mit, welche einen zeitkorrigierten Vergleich der Leitfähigkeiten der Dialyseflüssigkeit stromaufwärts und stromabwärts des Dialysators umfasst und von der Annahme ausgeht, dass die Durchlässigkeit der im Dialysator 2 befindlichen semipermeablen Membran für Harnstoff in einem festen Verhältnis zu deren Durchlässigkeit für Elektrolyte steht. Das Signal des Leitfähigkeitssensors 6 wird von Bluteinträgen wegen der vergleichbaren Elektrolytzusammensetzungen allenfalls geringfügig beeinflusst, wohingegen es bei Lufteinträgen zu temporären Abfällen in der Leitfähigkeit kommt.

Ferner umfasst das Dialysegerät einen in der Rücklaufleitung 4 angeordneten Luftabscheider 7, an dem ein in der Figur durch zwei Fühlerstifte dargestellter Levelsensor 8 angeordnet ist. Der Luftabscheider 7 dient grundsätzlich dazu, etwaige Lufteinträge in die Dialyseflüssigkeit zu überwachen, sodass solche etwaigen Lufteinträge bei der Bilanzierung der Dialyseflüssigkeit berücksichtigt werden können und die Ermittlung der dem Patienten entzogenen Flüssigkeitsmenge nicht verfälscht wird. Das Signal des Levelsensors 8 wird von etwaigen Bluteinträgen überhaupt nicht beeinflusst, wohl aber von Lufteinträgen.

Im Rahmen einer Variante des erfindungsgemäßen Konzepts zur Verhinderung von Fehlauslösungen werden das Signal des Leitfähigkeitssensors 6 und das Signal des Levelsensors 8 als Unterdrückungskriterien bei der Einleitung der Fehlerroutine berücksichtigt. Dies bedeutet, dass vor Einleiten der Fehlerroutine aufgrund einer Auffälligkeit im Signal des Blutlecksensors eine Kontrollabfrage stattfindet, wie sich die Signale des Leitfähigkeitssensors 6 und des Levelsensors 8 in zeitlicher Korrelation verändert hat.

Zeitliche Korrelation bedeutet dabei nicht Gleichzeitigkeit, sondern es wird ein zeitlicher Versatz berücksichtigt, welcher der Dauer entspricht, welche die Dialyseflüssigkeit bei gegebenem Fluss benötigt, um vom Blutlecksensor 5 zum Leitfähigkeitssensor 6 bzw. zum Luftabscheider 7 zu fließen.

Sofern an Leitfähigkeitssensor 6 und Levelsensor in zeitlicher Korrelation eine Auffälligkeit erkannt wird, welche auf einen Lufteintrag schließen lässt, wird die Fehlerroutine zumindest temporär unterdrückt und die Karenzzeit verlängert.

Anhand der Erfindung werden Fehlauslösungen sehr effektiv vermieden, während die Fehlerroutine bei tatsächlichen Bluteinträgen weiterhin zuverlässig ausgelöst wird und die Behandlungssicherheit somit in unverändertem Umfang gewährleistet werden kann.

## Patentansprüche

1. Dialysegerät mit einem Dialysator (2), einem extrakorporalen Blutkreislauf, einem Dialyselösungskreislauf (1) und einer Steuereinheit, wobei im Dialyselösungskreislauf stromabwärts des Dialysators (2) ein Blutlecksensor (5) angeordnet ist, wobei die Steuereinheit ausgebildet ist, eine Fehlerroutine einzuleiten, wenn das Signal des Blutlecksensors (5) einen Hinweis auf ein vermeintliches Blutleck zeigt, und wobei im Dialyselösungskreislauf (1) stromabwärts des Dialysators ferner ein weiterer Sensor angeordnet ist, wobei die Steuereinheit ausgebildet ist, das zeitlich mit dem Signal des Blutlecksensors (5) korrelierte Signal des weiteren Sensors (6) als Unterdrückungskriterium für die Einleitung der Fehlerroutine heranzuziehen und **gekennzeichnet dadurch, dass** im Dialyselösungskreislauf (1) stromabwärts des Dialysators (2) ein Luftabscheider (7) angeordnet ist und dass zwei weitere Sensoren vorgesehen sind, nämlich ein Leitfähigkeitssensor (6) sowie ein an dem Luftabscheider angeordneter Luftdetektor, wobei die Steuer-einheit ausgebildet ist, die zeitlich korrelierten Signale beider dieser weiteren Sensoren als Unterdrückungskriterien für die Einleitung der Fehlerroutine heranzuziehen.

2. Dialysegerät nach Anspruch 1, **dadurch gekennzeichnet, dass** es sich bei dem weiteren Sensor um einen Sensor handelt, der sich eines vom Blutlecksensor (5) unterschiedlichen Messverfahrens bedient und/oder der einen vom Blutlecksensor (5) unterschiedlichen Messparameter erfasst, wobei vorzugsweise vorgesehen ist, dass der Blutlecksensor (5) ausgebildet ist, dass dessen Signal durch Lufteinträge in die Dialyseflüssigkeit beeinflusst wird und dessen Signal durch Bluteinträge in die Dialyseflüssigkeit nicht oder weniger stark beeinflusst wird.

3. Dialysegerät nach Anspruch 1, **dadurch gekennzeichnet, dass** es sich bei dem Luftdetektor um einen Levelsensor (8), handelt.

4. Dialysegerät nach einem der vorhergehenden Ansprüche, **dadurch gekennzeichnet, dass** die Steuereinheit ausgebildet ist, die Fehlerroutine erst nach Ablauf einer Karenzzeit einzuleiten, während der die Auffälligkeit am Blutlecksensor (5) mehrmals oder fortlaufend erkannt wird.

5. Dialysegerät nach Anspruch 5, **dadurch gekennzeichnet, dass** die Steuereinheit ausgebildet ist, das Signal des weiteren Sensors während der Karenzzeit mehrmals oder fortlaufend abzufragen und diese Messwerte als Unterdrückungskriterien heranzuziehen.

6. Dialysegerät nach Anspruch 5 oder 6, **dadurch gekennzeichnet, dass** die Steuereinheit ausgebildet ist, die Karenzzeit zu verlängern, wenn am weiteren Sensor ein Signal erkannt wird, welches auf einen Störfaktor schließen lässt.

7. Dialysegerät nach einem der vorhergehenden Ansprüche, **dadurch gekennzeichnet, dass** die Fehlerroutine die Ausgabe eines Alarmsignals und/oder einen Behandlungsstopp umfasst.

8. Dialysegerät nach einem der vorhergehenden Ansprüche, **dadurch gekennzeichnet, dass** es sich bei dem Blutlecksensor (5) um einen optischen Sensor handelt, vorzugsweise um einen Sensor zur Durchführung einer Extinktionsmessung.

## Claims

1. Dialysis machine having a dialyzer (2), an extracorporeal blood circuit, a dialysis solution circuit (1), and a control unit, wherein a blood leak sensor (5) is arranged downstream of the dialyzer (2) in the dialysis solution circuit; wherein the control unit is configured to initiate an error routine when the signal of the blood leak sensor shows (5) an indication of a presumed blood leak; and wherein a further sensor is furthermore arranged downstream of the dialyzer in the dialysis solution circuit (1),
wherein
the control unit is configured to use the signal of the further sensor (6) correlated in time with the signal of the blood leak sensor (5) as a suppression criterion for the initiation of the error routine and **characterized in that** an air separator (7) is arranged downstream of the dialyzer (2) in the dialysis solution circuit; and **in that** two further sensors are provided, namely a conductivity sensor (6) and an air detector arranged at the air separator, wherein the control unit is configured to use the temporally correlated signals of both of these further sensors as suppression criteria for the initiation of the error routine.

2. Dialysis machine in accordance with claim 1, **characterized in that** the further sensor is a sensor that makes use of a measurement process differing from the blood leak sensor (5) and/or detects a measurement parameter differing from the blood leak sensor (5), with provision preferably being made that the blood leak sensor (5) is configured such that its signal is influenced by air intakes into the dialysis liquid and its signal is not influenced or is influenced less by blood intakes into the dialysis liquid.

3. Dialysis machine in accordance with claim 1, **characterized in that** the air detector is a level sensor (8).

4. Dialysis machine in accordance with one of the preceding claims, **characterized in that** the control unit is configured only to initiate the error routine after the end of a waiting period during which the abnormality at the blood leak sensor (5) is recognized several times or continuously.

5. Dialysis machine in accordance with claim 5, **characterized in that** the control unit is configured to poll the signal of the further sensor several times or continuously during the waiting period and to use these measurement values as suppression criteria.

6. Dialysis machine in accordance with claim 5 or 6, **characterized in that** the control unit is configured to extend the waiting period when a signal is recognized at the further sensor that allows a conclusion on a disruptive factor.

7. Dialysis machine in accordance with one of the preceding claims, **characterized in that** the error routine comprises the output of an alarm signal and/or a treatment stop.

8. Dialysis machine in accordance with one of the preceding claims, **characterized in that** the blood leak sensor (5) is an optical sensor, preferably a sensor for carrying out an extinction measurement.

## Revendications

1. Appareil de dialyse comprenant un dialyseur (2), un circuit sanguin extracorporel, un circuit de solution de dialyse (1) et une unité de commande, un capteur de fuite de sang (5) étant disposé dans le circuit de solution de dialyse en aval du dialyseur (2), l'unité de commande étant conçue pour lancer une routine de traitement d'erreurs quand le signal du capteur de fuite de sang (5) contient une indication d'une fuite de sang présumée, et un autre capteur étant en outre disposé dans le circuit de solution de dialyse (1) en aval du dialyseur,
dans lequel
l'unité de commande est conçue pour tenir compte du signal de l'autre capteur (6), corrélé dans le temps au signal du capteur de fuite de sang (5), comme critère de suppression pour le lancement de la routine de traitement d'erreurs et **caractérisé en ce qu'**un séparateur d'air (7) est disposé dans le circuit de solution de dialyse (1) en aval du dialyseur (2) et **en ce que** deux autres capteurs sont prévus, à savoir un capteur de conductivité (6) ainsi qu'un détecteur d'air disposé sur le séparateur d'air, l'unité de commande étant conçue pour tenir compte des signaux corrélés dans le temps de ces deux autres capteurs comme critères de suppression pour le lancement de la routine de traitement d'erreurs.

2. Appareil de dialyse selon la revendication 1, **caractérisé en ce que** l'autre capteur est un capteur qui utilise un procédé de mesure différent du capteur de fuite de sang (5) et/ou qui détecte un paramètre de mesure différent du capteur de fuite de sang (5), le capteur de fuite de sang (5) étant de préférence conçu de façon que son signal soit influencé par des entrées d'air dans le liquide de dialyse et que son signal ne soit pas ou soit moins fortement influencé par des entrées de sang dans le liquide de dialyse.

3. Appareil de dialyse selon la revendication 1, **caractérisé en ce que** le détecteur d'air est un capteur de niveau (8).

4. Appareil de dialyse selon l'une des revendications précédentes, **caractérisé en ce que** l'unité de commande est conçue pour lancer la routine de traitement d'erreurs uniquement après l'écoulement d'un délai de carence pendant lequel la particularité est reconnue plusieurs fois ou en continu au niveau du capteur de fuite de sang (5).

5. Appareil de dialyse selon la revendication 5, **caractérisé en ce que** l'unité de commande est conçue pour interroger le signal de l'autre capteur plusieurs fois ou en continu pendant le délai de carence et tenir compte de ces valeurs de mesure comme critères de suppression.

6. Appareil de dialyse selon la revendication 5 ou 6, **caractérisé en ce que** l'unité de commande est conçue pour prolonger le délai de carence si, sur l'autre capteur, un signal qui révèle un facteur perturbateur est reconnu.

7. Appareil de dialyse selon l'une des revendications précédentes, **caractérisé en ce que** la routine de traitement d'erreurs comprend l'émission d'un signal d'alarme et/ou un arrêt de traitement.

8. Appareil de dialyse selon l'une des revendications précédentes, **caractérisé en ce que** le capteur de fuite de sang (5) est un capteur optique, de préférence un capteur destiné à effectuer une mesure d'absorbance.
